# EUROPEAN PATENT SPECIFICATION

(11) **EP 1 494 692 B1**
(45) Date of publication and mention of the grant of the patent: **02.05.2007**
(21) Application number: 03745775.1
(22) Date of filing: 31.03.2003
(51) Int. Cl.: A61K 36/00, A61K 31/425, A61P 17/00

(54) **TOPICAL PHARMACEUTICAL COMPOSITIONS COMPRISING PROANTHOCYANIDINS, GLYCYRRHETINIC ACID AND TELMESTEINE FOR THE TREATMENT OF DERMATITIS**
TOPISCHE PHARMAZEUTISCHE ZUSAMMENSETZUNGEN MIT PROANTHOCYANIDINEN, GLYCYRRHETINIC SÄURE UND TELMESTEINE ZUR BEHANDLUNG VON DERMATITIS
COMPOSITIONS PHARMACEUTIQUES TOPIQUES COMPRENANT DES PROANTHOCYANIDINES, DE L'ACIDE GLYCYRRHETINIQUE ET TELMESTEINE POUR LE TRAITEMENT DES DERMATITES

(30) Priority: 09.04.2002 IT MI20020075
(43) Date of publication of application: 12.01.2005
(73) Proprietor: Sinclair Pharmaceuticals Limited, Godalming, Surrey GU7 2AB (GB)
(72) Inventor: MASTRODONATO, Marco, I-20121 Milano (IT); CIATTINI, Roberto, I 20131 Milano (IT)
(74) Representative: Jappy, John William Graham
(86) International application number: PCT/EP2003/003329
(87) International publication number: WO 2003/084553

(56) References cited:
- EP-A- 0 694 305
- EP-A- 1 256 335
- DE-A- 10 131 641
- US-A- 4 698 360
- PATENT ABSTRACTS OF JAPAN vol. 1995, no. 03, 28 April 1995 (1995-04-28) & JP 06 336421 A (KOSE CORP;OTHERS: 01), 6 December 1994 (1994-12-06) cited in the application
- EGGENSPERGER H: "ACTIVE COMPLEXES FOR SMOOTH SKIN NATURE COMBATS OXIDATIVE STRESS WIRKSTOFFKOMPLEXE FUER GLATTE HAUT NATUR GEGEN OXIDATIVEN STRESS" COSSMA: COSMETICS, SPRAY TECHNOLOGY, MARKETING, BRAUN FACHVERLAGE, KARLSRUHE, DE, vol. 2, no. 8, August 2001 (2001-08), pages 38-39, XP008016527 ISSN: 1439-7676

## Description

The present invention relates to pharmaceutical compositions for the topical administration comprising as active ingredients proanthocyanidins, glycyrrhetinic acid and telmesteine, in admixture with a suitable carrier.

The topical pharmaceutical compositions of the present invention are useful for the treatment of inflammatory conditions of the skin, such as atopic dermatitis, allergic contact dermatitis, seborrheic dermatitis, radiation dermatitis, psoriasis, xerosis and atopia as well as of mucosae and eye inflammatory conditions.

Dermatites are superficial skin inflammations, characterized by vesicles, erythema, edema, oozing, scaling or crusting lesions and intense itching. Various types of dermatitis exist: contact dermatitis, which can be caused by irritants with which the skin is in contact or by non-irritating substances to which the subject is allergic; atopic dermatitis, a chronic disease characterized by strong itching; seborrheic dermatitis, a scaling disease which mainly affect the face and scalp. In principle, the treatment consists in removing the offending agent, which however cannot in many cases be identified or removed. Treatment is therefore based on corticosteroids, which have however well known side-effects: they reduce the immune defenses, which can induce infections, mainly by fungi or Candida; suspension of the treatment should be gradual; they cannot be used during the acute oozing phase; a rebound effect may appear on stopping treatment. Furthermore, corticosteroids should not be used for prolonged treatments, particularly in children, since they can give rise to systemic effects.

In the case of seborrheic dermatitis, alternative treatments, based on hydrogenated vegetable oils or hydrophilic petrolatum, or medicated shampoos (based on zinc-pirithione, selenium sulfide, sulfur and the like) in are not resolutive.

In case of the mucosae inflammatory conditions, in particular of mouth, gingival, rectal, vaginal and eye mucosae, a number of topical treatments are available, including the use of steroidal or non-steroidal antiinflammatory agents, with the problems and side effects characteristics for these medicaments.

Different uses of proanthocyanidins have been described in the pharmaceutical and cosmetic fields. EP 0 694 305 discloses topical compositions of proanthocyanidins combined with coumarins (esculoside and the like) for the treatment of peripheral vasculopathies, such as bedsores, scars, couperose, varices and the like. US 5,470,874 describes a combination of proanthocyanidins and vitamin C for the topical use, as sunscreen, for stimulating collagen synthesis and for restoring damaged collagen. Finally, JP 6,336,421 relates to topical formulations of proanthocyanidins combined with anti-inflammatories, among which glycyrrhetinic acid and derivatives are cited, for the cosmetic use and against sunburns. However, to date the use of proanthocyanidins in the treatment of pathologies such as chronic dermatitis, seborrheic dermatitis and allergic dermatis has not been described.

Proanthocyanidins are widely diffused in a number of vegetable species. They are vegetable extracts containing bioflavonoids, with well-defined chemical profile, consisting for about 15% of dimers, about 20% of trimers and tetramers, and of small amounts of catechin and epicatechin. Proanthocyanidins exert both skin protecting action from the aggression by free radicals and restoration action of the damages to the skin structure through stimulation of collagen production. They also contain essential fatty acids similar to those of the skin hydrolipidic barrier, which contribute to keep said barrier intact. Finally, proanthocyanidins reduce the concentration of enzymes such as elastase, collagenase, hyaluronidase and beta-glucuronidase, which are responsible for the destruction of elastin, collagen and hyaluronic acid proteins. Therefore, proanthocyanidins are widely used in the pharmaceutical and cosmetic industries, thanks to their restoring, regenerating, nutrient and restructurant actions, which restores the skin elasticity and tonicity.

According to the present invention, the proanthocyanidins extracted from grape seeds and skin of *Vitis vinifera* are particularly preferred. Most preferred are the complexes of proanthocyanidins from *Vitis vinifera* with phospholipids, prepared according to the process disclosed in US 4,963,527.

18-β-Glycyrrhetinic acid, extracted from the roots of *Glycyrrhiza glabra,* is known to have antiinflammatory properties on the skin, in particular in case of bums and redness.

Telmesteine (N-carbethoxy-4-thiazolidinecarboxylic acid) exerts antiradicalic and protective action against the oxidizing agents responsible for skin damages, as well as inhibiting elastase and collagenase actions.

The topical pharmaceutical compositions of the present invention will contain the active ingredients in admixture with a suitable carrier, preferably a carrier rich in polyunsaturated fatty acids. According to the invention, suitable carriers comprise squalene, fatty acids, fatty acids esters, vegetable oils, natural or synthetic triglycerids. More preferably, suitable carriers comprise squalene, karité butter, octyl palmitate and oenothera oil.

In particular, karité butter (also known as shea butter) is a fat consisting of a mixture of saturated and unsaturated fats, extracted from the seeds of *Butirospermum parkii,* a tree from northern Africa, which is used in cosmetics thanks to its protective and softening actions, which make it particularly useful for sensitive skins as well as for skins which easily redden.

Oenothera oil (also known as evening primrose oil), extracted from the plant *Oenothera biennis,* is rich in essential polyunsaturated fatty acids, in particular γ-linolenic acid, indispensable for regenerating the skin and all cellular tissues.

According to a preferred embodiment, the pharmaceutical compositions of the invention will further contain compounds with antioxidizing activity, such as tocopherols and ascorbic acid or esters thereof, preferably tocopherol acetate and ascorbyl palmitate or tetrapalmitate, to further increase the protective effect on cell membranes and to slow down the oxidation of polyunsaturated fatty acids.

The compositions of the present invention may further contain other active ingredients, with complementary or anyway useful actions in the treatment of dermatosis.

Examples of said active ingredients are:
- salicylic acid, which exerts a keratolytic action useful for the treatment of seborrheic dermatitis;
- hyaluronic acid, which is useful for the treatment of radiation dermatitis due to its hydrating and healing action;
- alpha-bisabolol, one of the active principles present in chamomile essential oil (Matricaria flos), which has lenitive and anti-redness action;
- zinc pidolate, which exerts slightly astringent, emollient and lenitive actions, blocks and prevents the formation of free radicals thanks to its competitive action towards iron ions, and is active in the enzymatic processes of skin metabolism;
- allantoin which has astringent, slightly keratolytic and healing actions;
- moisturizers or wetting agents;
- piroctone olamine (octopirox), a known agent with antiseborrhoic activity.

Therefore, a further preferred embodiment relates to the pharmaceutical compositions of the invention also containing salicylic acid, for the treatment of seborrheic dermatitis.

A further preferred embodiment relates to the pharmaceutical compositions of the invention also containing hyaluronic acid, for the treatment of radiation dermatitis.

A further preferred embodiment relates to the pharmaceutical compositions of the invention also containing alpha-bisabolol and allantoin.

A further preferred embodiment relates to the pharmaceutical compositions of the invention also containing zinc pidolate.

The topical pharmaceutical compositions of the present invention can be in the form of cream, gel, lotion, suspension, spray, ointment, foam.

The topical pharmaceutical compositions of the present invention will contain the active ingredients in the following concentrations (w/w):
a) proanthocyanidins in the form of complexes with phospholipids: 0.01% to 1%;
b) glycyrrhetinic acid: 0.1 to 5%, preferably 1 to 2%;
c) telmesteine: 0.01% to 1%; and
   - carriers (squalene, karité butter, octyl palmitate and oenothera oil): 10-50%;
   - antioxidants (tocopherol acetate 0.5-5%; ascorbyl palmitate 0.01-0.1%);
   - salicylic acid 0.1-5%;
   - hyaluronic acid 0.1-10%;
   - alfa-bisabolol 0.1-3%;
   - zinc pidolate 0.01-1%;
   - allantoin 0.1-2%.

The daily dosage will be determined by the physician; by way of example, it will consist of one or more daily applications even for protracted times.

Some examples of formulations according to the present invention are shown hereinafter.

### Example 1

**CREAM**

| | |
|---|---|
| Complexes of proanthocyanidins from *Vitis vinifera* with phospholipids | 0.100 |
| Glycyrrhetinic acid | 0.800 |
| Telmesteine | 0.100 |
| Octyl palmitate | 7.000 |
| Pentylene glycol | 5.000 |
| Karité butter | 4.000 |
| Arachidyl alcohol, behenyl alcohol, C 12-20 alkylglucoside | 4.000 |
| Glyceryl stearate and glyceryl (100) OE stearate | 3.000 |
| Oenothera oil | 2.000 |
| Capriloyl glycine | 1.500 |
| Bisabolol | 1.200 |
| Vitamin E acetate | 1.000 |
| Carbomer | 0.700 |
| Octyl glycerin | 0.600 |
| Salicylic acid | 0.500 |
| Octopirox | 0.500 |
| Sodium hydroxide | 0.387 |
| Allantoin | 0.350 |
| Zinc pidolate | 0.100 |
| EDTA disodium salt | 0.08 |
| Ascorbyl palmitate | 0.05 |
| Propyl gallate | 0.02 |
| Water | 65.013 |
| Total | 100.000 |

### Example 2

**CREAM**

| | |
|---|---|
| Complexes of proanthocyanidins from Vitis vinifera with phospholipids | 0.100 |
| Glycyrrhetinic acid | 0.800 |
| Telmesteine | 0.100 |
| Octyl palmitate | 7.000 |
| Pentylene glycol | 5.000 |
| Karité butter | 4.000 |
| Arachidyl alcohol, behenyl alcohol, C 12-20 alkylglucoside | 4.000 |
| Glyceryl stearate and glyceryl (100) OE stearate | 3.000 |
| Squalene | 2.000 |
| Oenothera oil | 2.000 |
| Capriloyl glycine | 1.500 |
| Bisabolol | 1.200 |
| Vitamin E acetate | 1.000 |
| Carbomer | 0.700 |
| Octyl glycerin | 0.600 |
| Sodium hydroxide | 0.387 |
| Zinc pidolate | 0.100 |
| EDTA disodium salt | 0.08 |
| Ascorbyl palmitate | 0.05 |
| Propyl gallate | 0.02 |
| Water | 66.013 |
| Total | 100.000 |

### Example 3

**CREAM**

| | |
|---|---|
| Complexes of proanthocyanidins from Vitis vinifera with phospholipids | 0.100 |
| Glycyrrhetinic acid | 0.800 |
| Telmesteine | 0.010 |
| Dub po | 7.000 |
| Hydrolite-5 | 5.000 |
| Karitè butter | 4.000 |
| Montanov 202 | 4.000 |
| Arlacel 165 | 3.000 |
| Squalene ex | 2.000 |
| Oenothera oil | 2.000 |
| Lipacide C8G | 1.500 |
| Bisabolol | 1.200 |
| Vitamin E acetate | 1.000 |
| Carbopol ultrez 10 | 0.700 |
| Sensiva SC 50 | 0.600 |
| Octopirox | 0.500 |
| Sodium hydroxide drops P.P.A | 0.387 |
| Allantoin | 0.350 |
| Nipaguard DMDMH | 0.300 |
| Zincidone | 0.100 |
| EDTA disodium salt | 0.080 |
| Ascorbyl palmitate | 0.050 |
| Propyl gallate | 0.020 |
| Water | 65.303 |
| Total | 100.000 |

### Example 4

**CREAM**

| | |
|---|---|
| Complexes of proanthocyanidins from *Vitis vinifera* with phospholipids | 0.100 |
| Glycyrrhetinic acid | 0.800 |
| Telmesteine | 0.010 |
| Dub po | 7.000 |
| Hydrolite-5 | 5.000 |
| Karitè butter | 4.000 |
| Montanov 202 | 4.000 |
| Arlacel 165 | 3.000 |
| Squalene ex | 2.000 |
| Oenothera oil | 2.000 |
| Lipacide C8G | 1.500 |
| Bisabolol | 1.200 |
| Vitamin E acetate | 1.000 |
| Carbopol ultrez 10 | 0.700 |
| Sensiva SC 50 | 0.600 |
| Salicylic acid | 0.500 |
| Octopirox | 0.500 |
| Sodium hydroxide drops P.P.A | 0.465 |
| Allantoin | 0.350 |
| Nipaguard DMDMH | 0.300 |
| Zincidone | 0.100 |
| EDTA disodium salt | 0.080 |
| Ascorbyl palmitate | 0.050 |
| Propyl gallate | 0.020 |
| Water | 64.725 |
| Total | 100.000 |

### Example 5

**CREAM**

| | |
|---|---|
| Complexes of proanthocyanidins from *Vitis vinifera* with phospholipids | 0.100 |
| Glycyrrhetinic acid | 0.800 |
| Telmesteine | 0.010 |
| Dub po | 7.000 |
| Hydrolite-5 | 5.000 |
| Karitè butter | 4.000 |
| Montanov 202 | 4.000 |
| Arlacel 165 | 3.000 |
| Squalene | 2.000 |
| Oenothera oil | 2.000 |
| Lipacide C8G | 1.500 |
| Bisabolol | 1.200 |
| Vitamin E acetate | 1.000 |
| Carbopol ultrez 10 | 0.700 |
| Sensiva SC 50 | 0.600 |
| Octopirox | 0.500 |
| Sodium hydroxide drops P.P.A. | 0.387 |
| Allantoin | 0.350 |
| Nipaguard DMDMH | 0.300 |
| Zincidone | 0.100 |
| EDTA disodium salt | 0.080 |
| Ascorbyl palmitate | 0.050 |
| Hyaluronic acid sodium salt | 0.030 |
| Propyl gallate | 0.020 |
| Water | 65.273 |
| Total | 100.000 |

### Example 6

**CREAM**

| | |
|---|---|
| Complexes of proanthocyanidins from *Vitis vinifera* with phospholipids | 0.100 |
| Glycyrrhetinic acid | 2.000 |
| Telmesteine | 0.010 |
| Ethylhexyl palmitate | 9.000 |
| Butyrospermum parkii | 6.000 |
| Pentylene glycol | 5.000 |
| Butylene glycol | 3.000 |
| PEG-100 stearate | 1.500 |
| Glyceryl stearate | 1.500 |
| Capryloyl glycine | 1.500 |
| Arachidyl glucoside | 1.360 |
| Arachidyl alcohol | 1.320 |
| Behenyl alcohol | 1.320 |
| Bisabolol | 1.200 |
| Tocopheryl acetate | 1.000 |
| Carbomer | 0.700 |
| Ethylhexyl glycerin | 0.600 |
| Piroctone olamine | 0.500 |
| Sodium hydroxide | 0.387 |
| Allantoin | 0.350 |
| DMDM hydantoin | 0.300 |
| Sodium hyaluronate | 0.200 |
| Disodium EDTA | 0.080 |
| Tetrahexyidecyl ascorbate | 0.050 |
| Propyl Gallate | 0.020 |
| Water | 61.003 |
| Total | 100.000 |

### Example 7

**CREAM**

| | |
|---|---|
| Complexes of proanthocyanidins from *Vitis vinifera* with phospholipids | 0.100 |
| Glycyrrhetinic acid | 2.000 |
| Telmesteine | 0.010 |
| Ethylhxyl palmitate | 9.000 |
| Butyrospermum parkii | 6.000 |
| Pentylene glycol | 5.000 |
| Butylene glycol | 3.000 |
| PEG-100 stearate | 1.500 |
| Glyceryl stearate | 1.500 |
| Capryloyl glycine | 1.500 |
| Arachidyl glucoside | 1.360 |
| Arachidyl alcohol | 1.320 |
| Behenyl alcohol | 1.320 |
| Bisabolol | 1.200 |
| Salicylic acid | 1.000 |
| Tocopheryl acetate | 1.000 |
| Sodium hydroxide | 0.785 |
| Carbomer | 0.700 |
| Ethylhexylglycerin | 0.600 |
| Piroctone olamina | 0.500 |
| Allantoin | 0.3 50 |
| DMDM hydantoin | 0.300 |
| Disodium EDTA | 0.080 |
| Tetrahexyldecyl ascorbate | 0.050 |
| Propyl gallate | 0.020 |
| Water | 59.805 |
| Total | 100.000 |

### Example 8

**CREAM**

| | |
|---|---|
| Complexes of proanthocyanidins from *Vitis vinifera* with phospholipids | 0.100 |
| Glycyrrhetinic acid | 2.000 |
| Telmesteine | 0.010 |
| Ethylhexyl palmitate | 9.000 |
| Butyrospermum parkii | 6.000 |
| Pentylene glycol | 5.000 |
| Butylene glycol | 3.000 |
| PEG-100 stearate | 1.500 |
| Glyceryl stearate | 1.500 |
| Capryloyl glycine | 1.500 |
| Arachidyl glucoside | 1.360 |
| Arachidyl alcohol | 1.320 |
| Behenyl alcohol | 1.320 |
| Bisabolol | 1.200 |
| Tocopheryl acetate | 1.000 |
| Carbomer | 0.700 |
| Ethylhexylglycerin | 0.600 |
| Piroctone olamine | 0.500 |
| Sodium hydroxide | 0.387 |
| Allantoin | 0.350 |
| DMDM hydantoin | 0.300 |
| Sodium hyaluronate | 0.100 |
| Disodium EDTA | 0.080 |
| Tetrahexyldecyl ascorbate | 0.050 |
| Propyl gallate | 0.020 |
| Water | 61.103 |
| Total | 100.000 |

The compositions of the present invention showed very good tolerability. They do not contain allergenic substances, derivatives from animal sources (such as lanolin, beeswax, animal fat), preservatives (such as parabens, isothiazolones, phenol derivatives, and the like) which are often responsible for allergic contact dermatitis.

Therefore, thanks to the above mentioned characteristics, the compositions of the present invention are useful for the treatment of already existing skin allergic reactions, for the prevention of recurrent forms, and as adjuvants in the treatment of chronic diseases such as atopic dermatitis, allergic contact dermatitis, seborrheic dermatitis, radiation dermatitis, xerosis and atopia.

More particularly, the compositions of the present invention are useful in the treatment of conditions such as irritative and eczematous dermatitis, as moisturizers and lenitive agents for sensitive, delicate skin; in allergic irritations due to medicaments, detergents, solvents; in erythema due to excessive exposure to sun radiaitons; in case of insect stings, redness of various origin, post-shaving irritations, slight burns, skin hyper-reactivity; as normalizers after esthetic treatments, such as peeling with acid glycolic or laser-therapy.

The excellent tolerability of the compositions of the present invention makes them also suitable in pediatrics.

The present invention also relates to the use of a pharmaceutical composition comprising as active ingredients proanthocyanidins, glycyrrhetinic acid and telmesteine for the preparation of a topical medicament for the treatment of skin inflammations, in particular atopic dermatitis, allergic contact dermatitis, seborrheic dermatitis, radiation dermatitis, xerosis, psoriasis and atopia; and of mucosae inflammatory conditions, in particular of vaginal, rectal, eye gingival and buccal mucosae.

## Claims

1. A pharmaceutical composition for topical administration, comprising as active ingredients proanthocyanidins, glycyrrhetinic acid and telmesteine in admixture with a suitable carrier.

2. A pharmaceutical composition as claimed in claim 1, wherein the proanthocyanidins are used in the form of complexes with phospholipids.

3. A pharmaceutical composition as claimed in claim 1 or claim 2, wherein the proanthocyanidins are further used in combination with alpha-bisabolol.

4. A pharmaceutical composition as claimed in any preceding claim, wherein the proanthocyanidins are further used in combination with piroctone olamine.

5. A pharmaceutical composition as claimed in any preceding claim, wherein the proanthocyanidins are further used in combination with wetting agents and moisturisers.

6. A pharmaceutical composition as claimed in any preceding claim, in the form of a cream, gel, lotion, suspension, spray, ointment or foam.

7. A pharmaceutical composition as claimed in any preceding claim, in which the carrier comprises squalene, fatty acids, fatty acid esters, vegetable oils, natural or synthetic triglycerides.

8. A pharmaceutical composition as claimed in any preceding claim, in which the carrier comprises squalene, karitè butter, octyl palmitate and oenothera oil.

9. A pharmaceutical composition as claimed in any preceding claim, further comprising tocopherols, ascorbic acid or esters thereof.

10. A pharmaceutical composition as claimed in any preceding claim, comprising tocopherol acetate and ascorbyl palmitate or tetrapalmitate.

11. A pharmaceutical composition as claimed in any preceding claim, comprising salicylic acid.

12. A pharmaceutical composition as claimed in any preceding claim, comprising hyaluronic acid.

13. A pharmaceutical composition as claimed in any preceding claim, further comprising at least one compound selected from alpha-bisabolol, zinc pidolate, allantoin, piroctone olamine.

14. A pharmaceutical composition as claimed in any preceding claim, in which the following active ingredients are present in the following concentrations:
a) proanthocyanidins in the form of complexes with phospholipids: 0.01 % to 1%;
b) glycyrrhetinic acid: 0.1% to 5%;
c) telmesteine: 0.01% to 1%.

15. A composition as claimed in claim 14, in which glycyrrhetinic acid is present in concentrations ranging from 1 to 2%.

16. A pharmaceutical composition as claimed in any preceding claim, for use in therapy.

17. Use of a pharmaceutical composition as claimed in any of claims 1 to 15, in the manufacture of a medicament for the treatment of inflammatory conditions of the skin and mucosae.

18. Use according to claim 17, wherein the inflammatory condition is selected from atopic dermatitis, allergic contact dermatitis, seborrheic dermatitis, radiation dermatitis, xerosis, psoriasis, atopia, and inflammatory conditions of the vaginal, rectal, buccal and eye mucosae.

## Patentansprüche

1. Pharmazeutische Zusammensetzung zur topischen Verabreichung, die als wirksame Bestandteile Proanthocyanidine, Glycyrrhetinsäure und Telmestein im Gemisch mit einem geeigneten Träger umfasst.

2. Pharmazeutische Zusammensetzung nach Anspruch 1, wobei die Proanthocyanidine in Form von Komplexen mit Phospholipiden verwendet werden.

3. Pharmazeutische Zusammensetzung nach Anspruch 1 oder Anspruch 2, wobei die Proanthocyanidine zusätzlich in Kombination mit alpha-Bisabolol verwendet werden.

4. Pharmazeutische Zusammensetzung nach einem vorhergehenden Anspruch, wobei die Proanthocyanidine zusätzlich in Kombination mit Piroctonolamin verwendet werden.

5. Pharmazeutische Zusammensetzung nach einem vorhergehenden Anspruch, wobei die Proanthocyanidine zusätzlich in Kombination mit Benetzungsmitteln und Feuchtigkeitsmitteln verwendet werden.

6. Pharmazeutische Zusammensetzung nach einem vorhergehenden Anspruch, in Form einer Creme, eines Gels, einer Lotion, einer Suspension, eines Sprays, einer Salbe oder eines Schaums.

7. Pharmazeutische Zusammensetzung nach einem vorhergehenden Anspruch, in welcher der Träger Squalen, Fettsäuren, Fettsäureester, Pflanzenöle, natürliche oder synthetische Triglyceride umfasst.

8. Pharmazeutische Zusammensetzung nach einem vorhergehenden Anspruch, in welcher der Träger Squalen, Karitè-Butter, Octylpalmitat und Oenotheraöl umfasst.

9. Pharmazeutische Zusammensetzung nach einem vorhergehenden Anspruch, die zusätzlich Tocopherole, Ascorbinsäure oder deren Ester umfasst.

10. Pharmazeutische Zusammensetzung nach einem vorhergehenden Anspruch, die Tocopherolacetat und Ascorbylpalmitat oder - tetrapalmitat umfasst.

11. Pharmazeutische Zusammensetzung nach einem vorhergehenden Anspruch, die Salicylsäure umfasst.

12. Pharmazeutische Zusammensetzung nach einem vorhergehenden Anspruch, die Hyaluronsäure umfasst.

13. Pharmazeutische Zusammensetzung nach einem vorhergehenden Anspruch, die weiter mindestens eine Verbindung umfasst, die aus alpha-Bisabolol, Zinkpidolat, Allantoin, Piroctonolamin ausgewählt ist.

14. Pharmazeutische Zusammensetzung nach einem vorhergehenden Anspruch, in welcher die nachfolgenden wirksamen Bestandteile in den folgenden Konzentrationen vorliegen:
a) Proanthocyanidine in Form von Komplexen mit Phospholipiden: 0,01% bis 1%;
b) Glycyrrhetinsäure: 0,1% bis 5%;
c) Telmestein: 0,01% bis 1%.

15. Zusammensetzung Anspruch 14, in welcher Glycyrrhetinsäure in Konzentrationen im Bereich von 1 bis 2% vorliegt.

16. Pharmazeutische Zusammensetzung nach einem vorhergehenden Anspruch zur Verwendung in der Therapie.

17. Verwendung einer pharmazeutischen Zusammensetzung nach einem der Ansprüche 1 bis 15 zur Herstellung eines Medikaments zur Behandlung von entzündlichen Erkrankungen der Haut und der Schleimhäute.

18. Verwendung nach Anspruch 17, wobei die entzündliche Erkrankung aus atopischer Dermatitis, allergischer Kontaktdermatitis, seborrheischer Dermatitis, Strahlungsdermatitis, Xerosis, Psoriasis, Atopie und entzündlichen Erkrankungen der Vaginal-, Rektal-, Bukkal- und Augenschleimhäute ausgewählt ist.

## Revendications

1. Composition pharmaceutique pour une administration topique, comprenant en tant qu'ingrédients actifs des proanthocyanidines, de l'acide glycyrrhétinique et de la telmestéine en mélange avec un support approprié.

2. Composition pharmaceutique selon la revendication 1, dans laquelle les proanthocyanidines sont utilisées sous la forme de complexes avec des phospholipides.

3. Composition pharmaceutique selon la revendication 1 ou la revendication 2, dans laquelle les proanthocyanidines sont en outre utilisées en combinaison avec l'alpha-bisabolol.

4. Composition pharmaceutique selon l'une quelconque des revendications précédentes, dans laquelle les proanthocyanidines sont en outre utilisées en combinaison avec la piroctone olamine.

5. Composition pharmaceutique selon l'une quelconque des revendications précédentes, dans laquelle les proanthocyanidines sont en outre utilisées en combinaison avec des agents mouillants et des hydratants.

6. Composition pharmaceutique selon l'une quelconque des revendications précédentes, sous la forme d'une crème, d'un gel, d'une lotion, d'une suspension, d'un spray, d'une pommade ou d'une mousse.

7. Composition pharmaceutique selon l'une quelconque des revendications précédentes, dans laquelle le support comprend un squalène, des acides gras, des esters d'acides gras, des huiles végétales, des triglycérides naturels ou synthétiques.

8. Composition pharmaceutique selon l'une quelconque des revendications précédentes, dans laquelle le support comprend un squalène, du beurre de karité, du palmitate d'octyle et de l'huile d'oenothéra.

9. Composition pharmaceutique selon l'une quelconque des revendications précédentes, comprenant en outre des tocophérols, de l'acide ascorbique ou des esters de celui-ci.

10. Composition pharmaceutique selon l'une quelconque des revendications précédentes, comprenant de l'acétate de tocophérol et du palmitate ou du tétrapalmitate d'ascorbyle.

11. Composition pharmaceutique selon l'une quelconque des revendications précédentes, comprenant de l'acide salicylique.

12. Composition pharmaceutique selon l'une quelconque des revendications précédentes, comprenant de l'acide hyaluronique.

13. Composition pharmaceutique selon l'une quelconque des revendications précédentes, comprenant en outre au moins un composé choisi parmi l'alpha-bisabolol, le pidolate de zinc, l'allantoïne, la piroctone olamine.

14. Composition pharmaceutique selon l'une quelconque des revendications précédentes, dans laquelle les ingrédients actifs suivants sont présents aux concentrations suivantes :
a) proanthocyanidines sous la forme de complexes avec des phospholipides : de 0,01 % à 1 % ;
b) acide glycyrrhétinique : de 0,1 % à 5 % ;
c) telmestéine : de 0,01 % à 1 %.

15. Composition selon la revendication 14, dans laquelle l'acide glycyrrhétinique est présent à des concentrations dans l'intervalle de 1 à 2 %.

16. Composition pharmaceutique selon l'une quelconque des revendications précédentes, pour son utilisation en thérapie.

17. Utilisation d'une composition pharmaceutique selon l'une quelconque des revendications 1 à 15, dans la fabrication d'un médicament pour le traitement des états inflammatoires de la peau et des muqueuses.

18. Utilisation selon la revendication 17, dans laquelle l'état inflammatoire est choisi parmi la dermite atopique, l'eczéma de contact allergique, la parakératose séborrhéïque, la dermite due à des radiations, le xérosis, le psoriasis, l'atopie et les états inflammatoires des muqueuses vaginale, rectale, buccale et oculaire.
